Europäisches Patentamt

European Patent Office

Office européen des brevets

(19)

(11) Publication number: **0 019 626**
**B1**

(12) **EUROPEAN PATENT SPECIFICATION**

(45) Date of publication of patent specification: **11.01.84**

(51) Int. Cl.³: **A 61 L 9/00, A 61 L 9/04**

(21) Application number: **79901367.7**

(22) Date of filing: **16.10.79**

(86) International application number:
**PCT/US79/00847**

(87) International publication number:
**WO 80/00792 01.05.80 Gazette 80/10**

(54) **EVAPORATIVE DISPENSER.**

<table>
<tr><td>

(30) Priority: **18.10.78 US 952386**
**13.08.79 US 65848**

(43) Date of publication of application:
**10.12.80 Bulletin 80/25**

(45) Publication of the grant of the patent:
**11.01.84 Bulletin 84/2**

(84) Designated Contracting States:
**CH DE FR GB NL SE**

(56) References cited:
**CA - A - 487 281**
**FR - A - 2 227 877**
**US - A - 1 439 319**
**US - A - 2 202 235**
**US - A - 2 759 713**
**US - A - 2 828 953**
**US - A - 3 194 426**
**US - A - 3 225 951**
**US - A - 3 348 828**
**US - A - 3 356 244**
**US - A - 3 633 881**
**US - A - 3 804 592**

</td><td>

(73) Proprietor: **GEORGIA-PACIFIC CORPORATION**
**133 Peachtree Street, N.E.**
**Atlanta Georgia 30303 (US)**

(72) Inventor: **DeLUCA, Raymond F.**
**15 Whittaker Street**
**Stamford, CT 06902 (US)**

(74) Representative: **Kalkoff, Heinz-Dieter, Dipl.-Ing.**
**Patentanwälte Wenzel & Kalkoff Ruhrstrasse 26**
**Postfach 2448**
**D-5810 Witten (DE)**

(56) References cited:
**US - A - 3 970 250**
**US - A - 3 990 848**
**US - A - 3 993 444**
**US - A - 4 003 967**
**US - A - 4 035 451**
**US - A - 4 059 422**
**US - A - 4 113 129**
**US - A - 4 165 812**
**US - A - 4 166 087**
**US - A - 4 173 604**

</td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Evaporative dispenser

The present inventions relates to a method of emitting a vaporized material into the ambient at a controlled, substantially uniform rate from a substantially open reservoir containing an exposed liquid quantity of said material, said quantity being replenished from a replaceable, air-tight supply container of fresh liquid material removably supported above and in close proximity to said reservoir, thereby forming a small gap between the body of the said container and said reservoir, said reservoir and said container being located within a housing having air inlet means and air outlet means and relates also to a dispenser for emitting a vaporized material into the ambient at a controlled, substantially uniform rate comprising a housing having air inlet means and air outlet means, a substantially open reservoir within said housing for containing an exposed quantity of liquid material to be vaporized, an air-tight supply container for continually and automatically replenishing material evaporated from said reservoir, support means for removably supporting the supply container within said housing, and air circulating means for effecting a forced flow of ambient air through said dispenser.

Prior art freshener devices are of many different types. These may be classified into two broad categories, namely those which rely upon natural convection of room air to evaporate and diffuse air freshening material throughout a room, and those which rely upon forced air circulation (for example, by means of a motor-driven fan) to enhance the evaporative and distributive process. Air freshener devices which fall within each of these two categories may dispense evaporated material from any one of a number of different types of sources. These include liquid-containing reservoirs, paste or gel containing and impregnated porous media.

Impregnated media devices and gel or paste devices suffer the disadvantage that the quantity of air refreshening material remaining therein is difficult to ascertain. This is especially true in the case of impregnated media devices where it is impossible to visually observe of the amount of material remaining in the media. With devices of these types, the material supplied will often be depleted and the device will operate ineffectively for a period of time until the depletion is discovered and the supply renewed. Alternatively, the supply is often renewed prior to depletion, therefore resulting in material waste.

There are other air freshener devices which function as liquid evaporators and which make use of a wick structure immersed in a reservoir containing a quantity of liquid material to be dispensed into the ambient. Substantially all of the vaporation of this material occurs from the surface of the wick itself, and the liquid supply is replenished only infrequently. These devices suffer the disadvantage that the amount of deodorizer, that is vaporized gradually diminishes as the supply in the reservoir decreases. This results in an excessive release of fragrance at the start, and an insufficient release towards the end. This is due to the dilution of the concentration of the volatile fragrances as time passes, since the reservoir is always open, and the crusting over of the vaporization surfaces with the passage of time.

Air freshener devices without a motor-driven fan have been designed which overcome the irregular evaporation by using a liquid reservoir which often includes a material supply container or bottle for automatically replenishing the material evaporated from the reservoir. Since these devices rely upon natural convection, the volativity of the material has to be rather high in order to guarantee a certain evaporation. However, if such a device is submitted to a draught the evaporation is so rapid that the fragrance may become too strong and that the period for the need of replenishing is rather short due to this waste.

Accordingly it is an object of the present invention to propose an evaporative dispenser and a method for emitting vaporized material into the ambient wherein the vaporization surfaces are automatically and frequently renewed to prevent encrustation thereof and a controlled substantially uniform rate of vaporized material into the ambient is guaranteed.

As to the method the invention proposes to fulfill this object by the steps of producing and directing a forces flow of ambient air within said housing through said gap past and close to the surface of the liquid in the reservoir to effect vaporization of said liquid from said surface and directly entrain the vaporized material in said air flow; and continually and automatically replenishing material evaporated from said reservoir by discharging liquid material directly onto the surface of the liquid in the reservoir from said container to renew said surface and prevent stagnation thereof.

As to the dispenser the invention proposes to fulfill this object that air chanelling means is provided for directing said flow of air past and close to the surface of a liquid in said reservoir to effect vaporization of said liquid from said surface and directly entrain the vaporized material in said air-flow, and said supply container discharges fresh liquid directly onto the surface of the liquid in said reservoir to renew said surface and prevent stagnation thereof.

By producing and directing a forced flow of ambient air past and close to the surface of the liquid in the reservoir there is no alteration in the flow rate of the air-flow so that the volativity of the medium in the reservoir can be easily matched to this air-flow. In this way a steady flow of fragrance is discharged when the method or the device of the present invention is used. Foreseeable working periods are achieved before replenishing becomes due. Since the vaporization surfaces are continually and automatically renewed there is no possibility of crusting over which would lead to an unsteady flow of fragrance into the ambient air.

Preferably, the supply container is supported above the reservoir and its downwardly directed outlet is in sealed fluid communication with the liquid surface of the reservoir. When the forced flow of ambient air is directed onto the surface the level of the material in the reservoir drops due to depletion and the liquid seal between the container outlet and the liquid surface is broken, allowing air to enter the container and causing the liquid to spill out until the liquid seal is again effective.

Preferably, the exterior of the supply container is provided with a protrusion which cooperates with the housing of the dispenser to define an airway for directing the forced flow towards the reservoir. If the top wall of the container has a sloping portion which slopes downwardly and away from the central support means of the container, the air-passage being defined partly by the sloping portion, has a decreasing cross-section for the air-flow which causes it to accelerate, thereby resulting in a drop in static air pressure which enhances vaporization of the material from the reservoir.

A motor-driven fan for generating the forced air flow, the material supply bottle and the material reservoir are all compactly stacked within the dispenser housing. The fan establishes an entraining flow of air through the housing and across the reservoir. The supply container is preferably formed with a recess in which a battery nests when the supply container is in position within the cavity. The battery is retained in a separate support and need not to be removed when the supply container is to be replaced. In a preferred embodiment of the present invention a viewing window formed in the housing is located in registry with a transparent portion of the reservoir so that the level of material within the reservoir can be ascertained without opening the housing.

Preferred embodiments of the invention as set forth in the accompanying drawings are described in greater detail below; the drawings comprise

Figure 1 is a side elevational view of the dispenser with the housing shown in section;

Figure 2 is a front elevational view of the dispenser with the housing shown in section;

Figure 3 is a plan view of the dispenser with the housing shown in section;

Figure 4 is a vertical sectional view of the dispenser;

Figure 5 is a horizontal sectional view of the dispenser taken along line 5—5 of Figure 2;

Figure 6 is a front elevational view of the backplate of the dispenser;

Figure 7 is a horizontal sectional view of the same taken along 7—7 of Figure 6;

Figure 8 is a partial vertical sectional view of the same taken along line 8—8 of Figure 6;

Figure 9 is a front elevational view of the chassis of the dispenser;

Figure 10 is a side elevational view of the same;

Figure 11 is a vertical sectional view of the same taken along line 11—11 of Figure 9;

Figure 12 is a horizontal sectional view of the same taken along line 12—12 of Figure 9;

Figure 13 is a horizontal sectional view of the same taken along 13—13 of Figure 9;

Figure 14 is a horizontal sectional view of the same taken along line 14—14 of Figure 9;

Figure 15 is a horizontal sectional view of the assembled backplate and chassis taken along line 15—15 of Figures 8 and 9;

Figure 16 is a partial vertical sectional view of the assembled chassis and reservoir support taken along line 16—16 of Figures 9 and 17, and showing the relative position of the supply container;

Figure 17 is a front elevational view of the reservoir assembly of the dispenser;

Figure 18 is a plan view of the same;

Figure 19 is a horizontal sectional view of the chassis taken along line 19—19 of Figure 16, and showing the relative positions of battery, reservoir support and supply container;

Figure 20 is a side elevational view of one spring lead for connecting the battery to the motor, shown in its relaxed state;

Figure 21 is a front elevational view of the same;

Figure 22 is a front elevational view of the other spring lead for connecting the battery to the motor, shown in its relaxed state;

Figure 23 is a side elevational view of the same;

Figure 24 is a perspective view of one embodiment of the supply container used in the dispenser;

Figure 25 is a bottom view of the same;

Figure 26 is a side elevational view of an alternative embodiment of the invention;

Figure 27 is a sectional view of the same taken along line 28—28 of Figure 26; and

Figures 28 through 30 depict an alternative embodiment of the supply container of the invention.

Description of Preferred Embodiments

The dispenser of the present invention may take the form of an air freshener device which is adapted for wall mounting in restrooms or the like. The exterior appearance of this air freshener is substantially identical to that of the liquid soap dispenser disclosed in US—A—4,036,406, and is intended for companion use therewith. The air freshener comprises seven subassemblies designated by the following series of reference numerals:

100 backplate
200 chassis
300 housing
400 motor and fan assembly
500 battery and lead assembly
600 reservoir assembly
700 supply container.

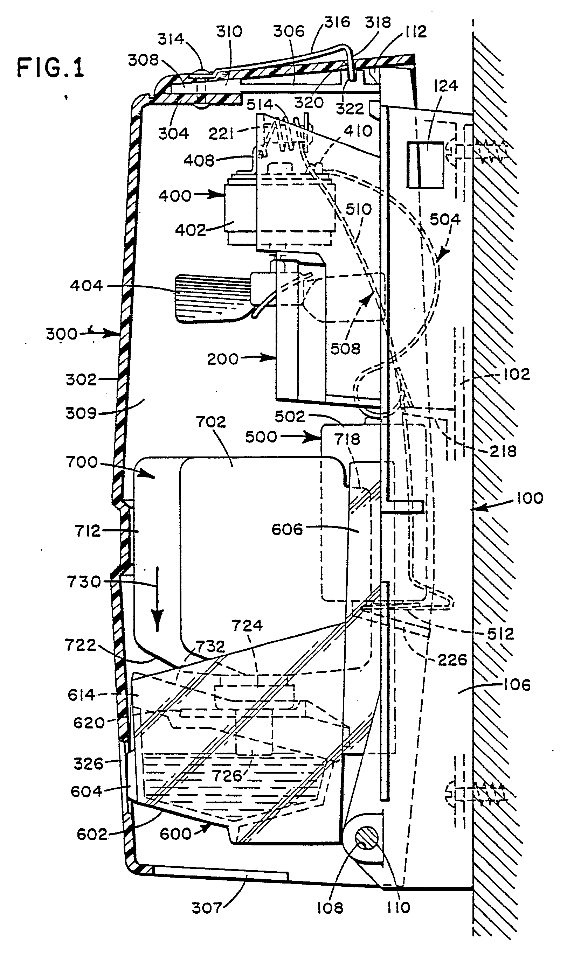

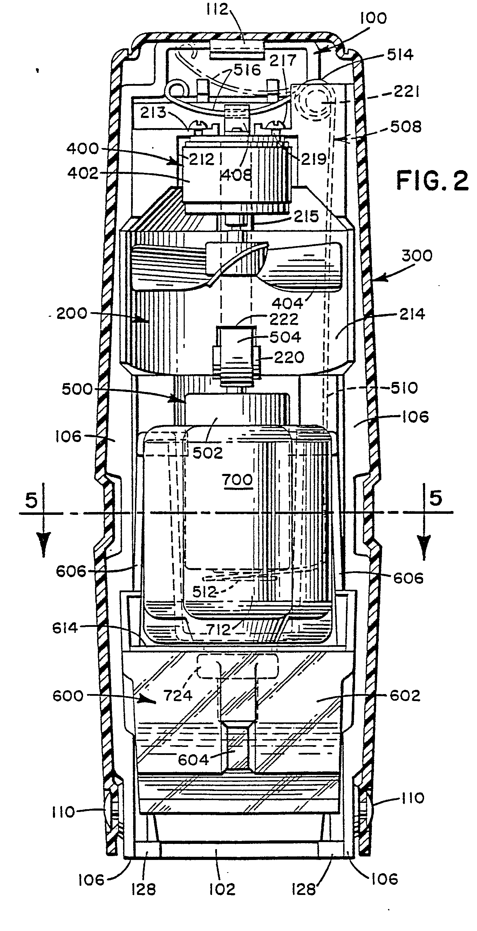

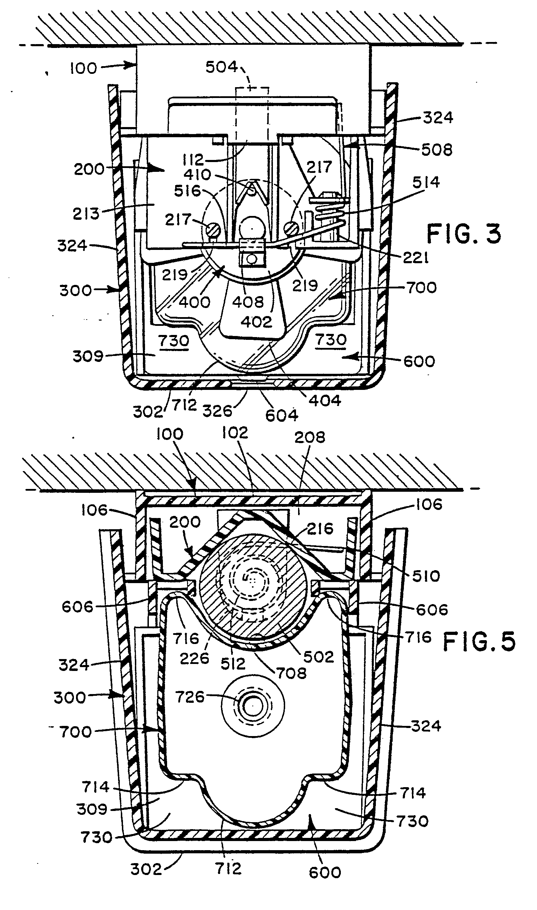

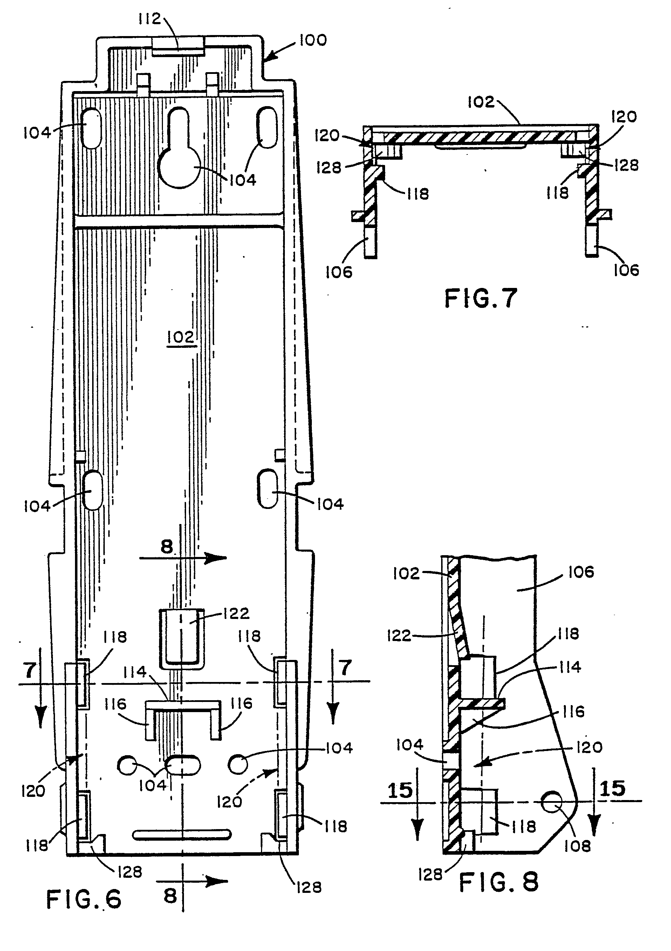

Referring to Figures 1 through 5, these subassemblies are generally related as follows. Backplate 100 is adapted to be mounted to a wall and supports chassis 200 and housing 300. Motor and fan assembly 400, battery and lead assembly 500, and reservoir assembly 600 are all secured to chassis 200. Supply container 700 is supported on reservoir assembly 600.

Backplate

Referring to Figures 1 through 8, backplate 100 is a molded plastic subassembly comprising a rear wall 102 having a plurality of mounting holes 104 through which mounting screws may be installed. Side flanges 106 extend forwardly from rear wall 102 and contain holes 108 through which pivot pins 110 pass for pivotally mounting housing 300 to the backplate. A protruding tab 112 extends forwardly from rear wall 102 for engaging the upper portion of housing 300.

A protruding seat 114 extends forwardly from rear wall 102 and is reinforced by webs 116. Guides 118 extend inwardly from side flanges 106 to define tapered, vertical channels 120 bounded in the rear by rear wall 102, on the sides by side flanges 106, at the front by guides 118 and at the bottom by inwardly extending stops 128. Channels 120 slidably receive and support mating structure of chassis 200, as explained more fully below. The taper of channel 120 provides a wedging action between the chassis structure and the backplate. A slanted tab 122 protrudes between channels 120 and is deflected rearwardly by the insertion of the chassis, and then snaps forwardly to engage a mating shoulder of the chassis when the chassis is bottomed in channels 120 against stops 128. Apertures 124 formed in side flanges 106 serve as two of several inlets for air entering the dispenser.

Chassis

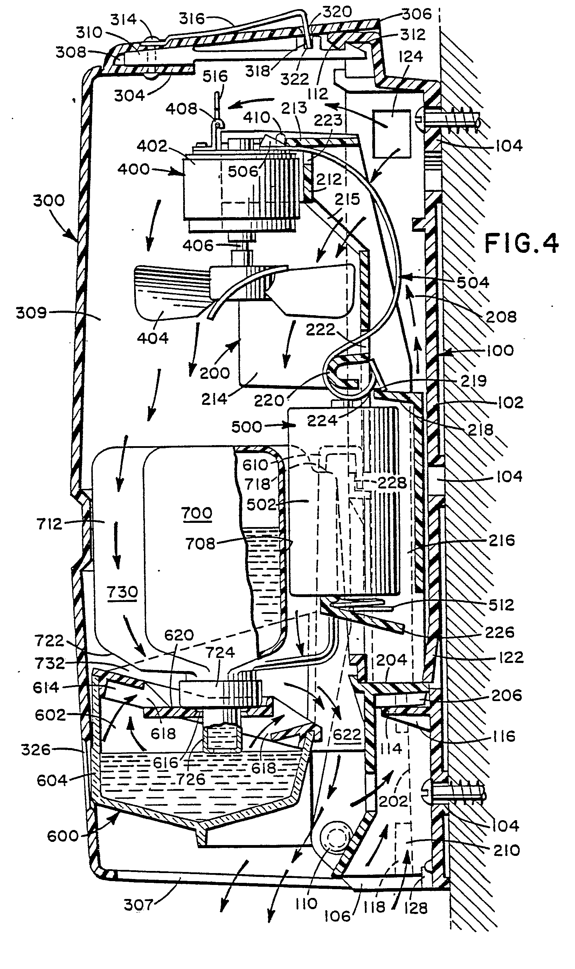

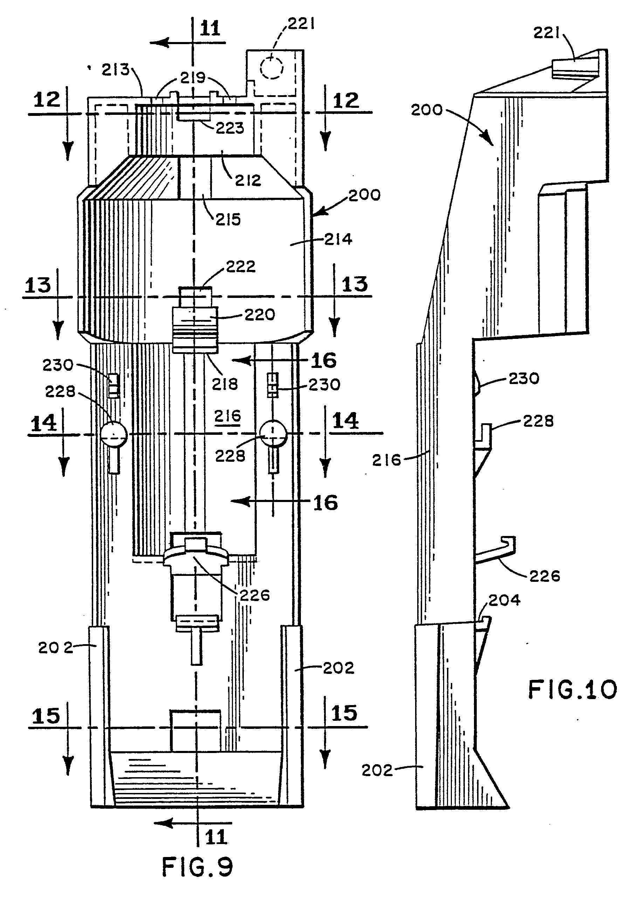

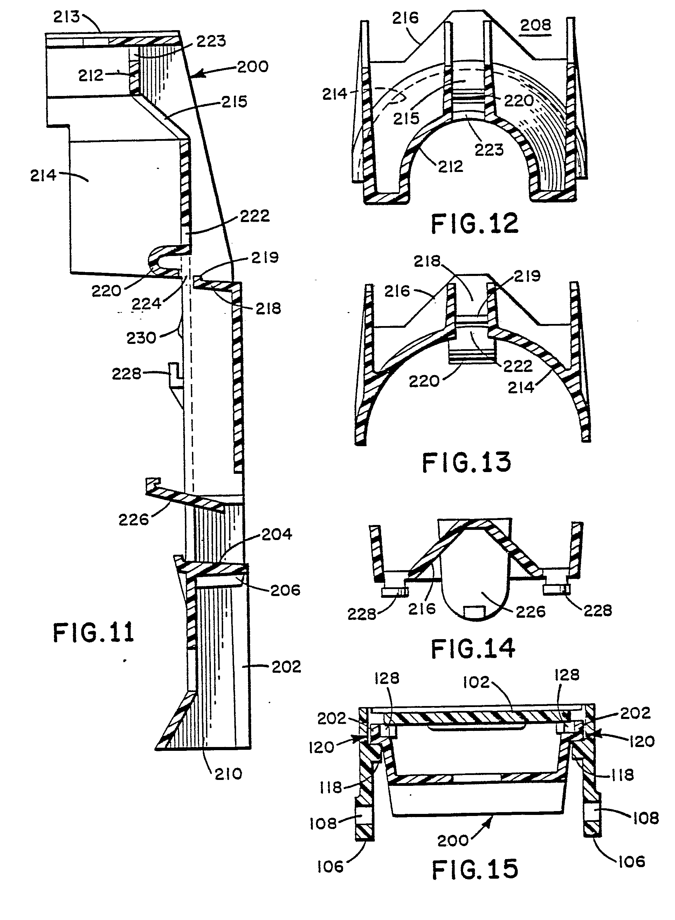

Referring to Figures 1 through 5 and 9 through 15, chassis 200 is also a molded plastic subassembly and comprises a generally vertically extending, contoured structure having a pair of tapered, laterally extending mounting rails 202 formed near the bottom thereof. A rearwardly extending tab 204 is provided with a bottom reinforcing flange 206. Chassis 200 is supported on backplate 100 by inserting the lower ends of mounting rails 202 into channels 120 behind upper tabs 118 and pressing the chassis downwardly until it bottoms against stops 128, as illustrated in Figure 15. At this point, flange 206 is firmly seated against seat 114, and tab 122, having been deflected rearwardly by tab 204, snaps forwardly to firmly engage the upper surface of tab 204 (see Figure 4). The chassis is thus firmly wedged and locked in place on the backplate and is supported over approximately the bottom one-quarter of its height. A longitudinal channel 208 is formed between chassis 200 and backplate 100. This channel, with its open bottom 210, serves as an additional air inlet. Air is drawn upwardly through channel 208 to the space above the motor and fan assembly.

An upper arcuate recess 212 cradles the motor of the fan and motor assembly 400. The motor is secured to horizontal flange 213 by means of screws 217 which extend through slots 219 formed in flange 213. A flange-mounted post 221 provides support for one end of one of the spring leads connected to the motor. A lower, larger arcuate recess 214 defines a partial shroud for the fan. An aperture 215 formed between arcuate recesses 212 and 214 communicates with air channel 208 and air inlet apertures 124.

A generally V-shaped battery tray 216 is formed below recess 214. Battery tray 216 comprises an upper flange 218 having a lip 219 which, in cooperation with nose portion 220 and apertures 222 and 224, accommodates the end of one of the spring leads which electrically connect the battery to the motor. The other end of this lead is trained through aperture 223. Battery tray 216 further comprises a lower hooked shelf 226 which supports the end of the other spring lead.

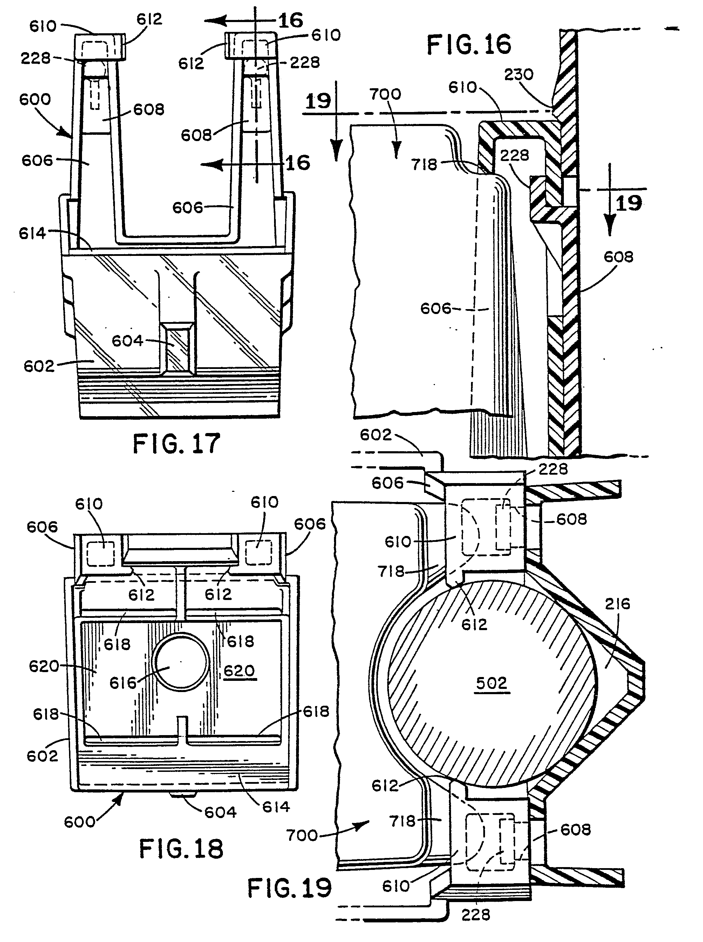

Forwardly extending hooks 228 cooperate with protrusions 230 to support and lock a hanging arm of reservoir assembly 600 (see Figure 16). An additional lower shelf 232 provides support for a lower portion of reservoir assembly 600 (see Figure 4).

Housing

Referring to Figures 1 through 5, housing 300 comprises a front wall 302, side walls 324, a top flange 304 and a top wall 306. The bottom of the housing is substantially open to form an outlet 307 for air and entrained vaporized air freshening material. The housing walls and other internal structure define a container receiving cavity 309.

Flange 304 and wall 306 define a space 308 therebetween for the reception of a locking member 310 which has a hooked nose portion 312 adapted to engage locking tab 112. Locking member 310 is retained in position by means of a rivet 314. Rivet 314 also secures a releasing spring lever 316 to top wall 306. Spring lever 316 has a leg portion 318 which extends through a hole 320 in top wall 306 to engage a seat 322 formed on locking member 310. By manually depressing spring lever 316, locking member 310 is deflected downwardly to uncouple nose portion 312 from locking tab 112. Side walls 324 are pivoted to side flanges 106 of backplate 100 by means of pins 110 which extend through apertures 108 formed in side flanges 106. Housing 300 may therefore be unlatched at the top by manually depressing spring lever 316 and swung downwardly about pins 110 to reveal the interior of the dispenser.

A viewing window 326 is formed in the lower portion of front wall 302. This viewing window is in registry with a transparent portion of the reservoir assembly 600. This permits the material level within the reservoir to be monitored without having to dismantle any portion of the dispenser.

## Motor and Fan Assembly

Referring to Figures 1 through 5, motor and fan assembly 400 comprises a motor 402 secured to chassis 200 as described above. A multi-bladed fan 404 is secured to the shaft 406 of motor 402. The blades of fan 404 have a pitch and direction of rotation such that an air flow is established downwardly through the housing, thereby drawing air into the housing through apertures 124, and opening 210. One terminal of motor 402 comprises a hook member 408 which retains one end of one of the spring leads extending from the battery. The other motor terminal comprises a post 410 which is engaged by the bifurcated end of the other spring lead.

## Battery and Lead Assembly

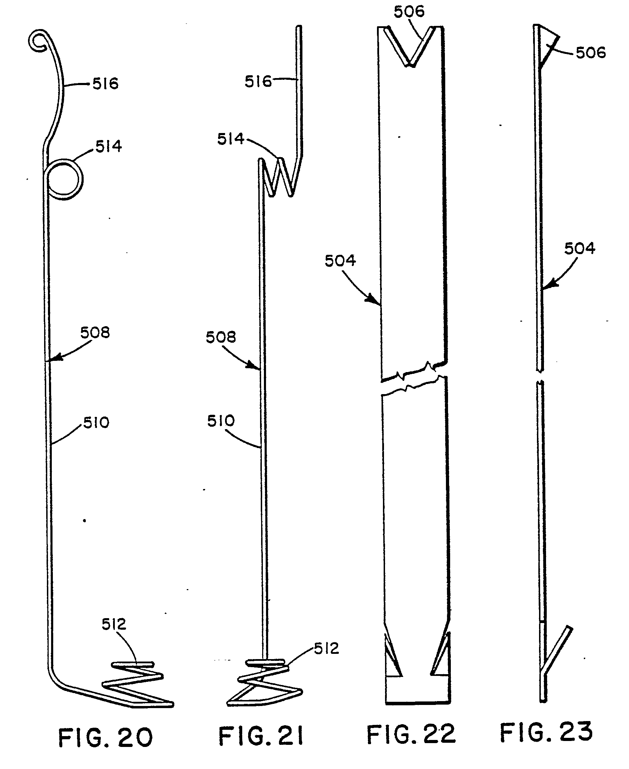

Referring to Figures 1 through 5, 19 and 21 through 23, battery and lead assembly 500 comprises a replaceable cylindrical dry cell battery 502 which is frictionally and resiliently supported in battery tray 216 of chassis 200 between the ends of two spring leads. Upper spring lead 504 comprises a flat strip of spring metal in its relaxed (uninstalled) state (see Figures 22 and 23) having a bifurcated tip 506 which engages motor terminal 410. The other end of spring lead 504 has a pair of prongs which engage behind lip 219 of flange 218. As described above, the spring lead is trained through aperture 224, around nose portion 220, through aperture 222 and through aperture 223 in engagement with motor terminal 410.

The other spring lead 508 comprises a spring wire of round cross-section having a generally straight center portion 510 in its relaxed state (see Figures 20 and 21). The lower end of spring lead 508 comprises a spiral coil 512 which is retained in hooked shelf 226 of chassis 200. The opposite end of this spring lead comprises a helical coil 514 which is trained about post 221 of chassis 200. A tip 516 which extends laterally of helical coil 514 is biased upwardly by coil 514 but is retained by hook terminal 408. The motor may be shut off by manually depressing tip 516 and moving it laterally out of engagement with hook terminal 408.

## Reservoir Assembly

Referring to Figures 1, 2, 4 and 16 through 19, reservoir assembly 600 comprises a reservoir 602 for containing a quantity of liquid odorant or deodorant material to be evaporated. Reservoir 602 may have a transparent portion 604 formed at the front thereof in registry with viewing window 326, but preferably it is made completely of transparent plastic. Upstanding arms 606 are integrally formed with reservoir 602 and serve to support the entire assembly on hooks 228 of chassis 200 by means of rearwardly facing apertures 608. Arms 606 carry lateral projections 610 near their upper ends which overlie and engage shoulders formed on the supply container. Inwardly extending tabs 612 are carried by lateral projections 610 and serve to hold battery 502 in position in its recess 216 (see Figure 19).

Reservoir 602 supports a lid 614 having a circular central aperture 616 and a plurality of louvers or slots 618. The central flat portion 620 which surrounds aperture 616 acts as a supporting surface for the supply container. Lid 614 slopes gradually downwardly towards backplate 100, terminating ahead of chassis 200 to define a channel 622 through which air with entrained vaporized material will pass on its way to outlet 307.

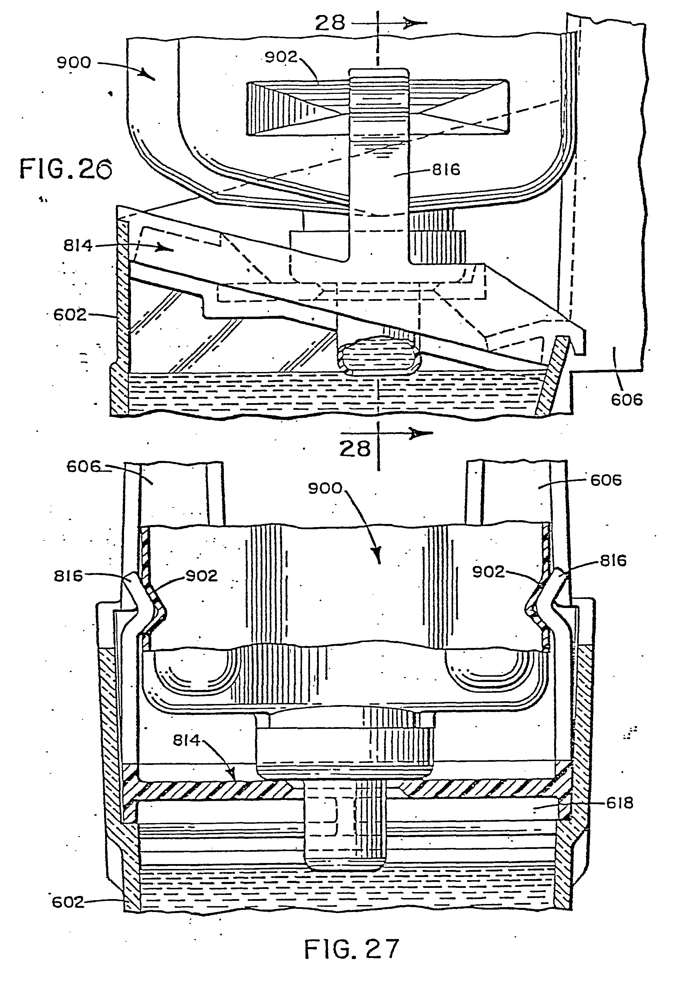

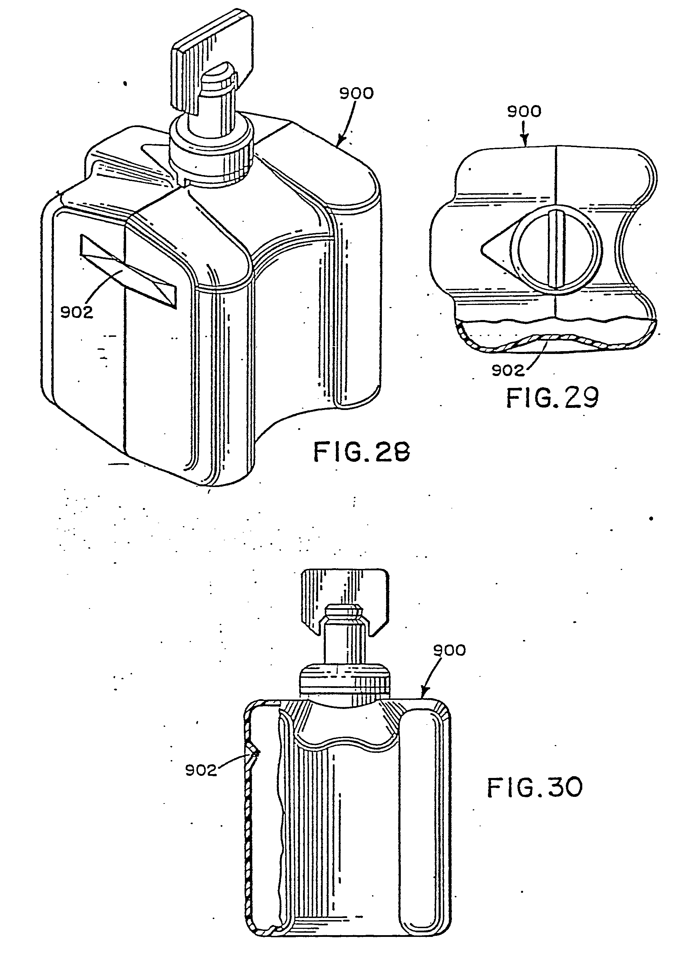

An alternative embodiment of lid 614 is illustrated in Figures 26 and 27. This lid is adapted to support the type of supply container illustrated in Figures 28, 29 and 30 and described below. Lid 814 is provided with an opposed pair of snap tabs 816 which cooperate with the dimples formed in container 900 to retain it in position on the lid. The resiliency of snap tabs 816 and that of the container walls both contribute to this snap-acting retention of the container.

## Supply Bottle

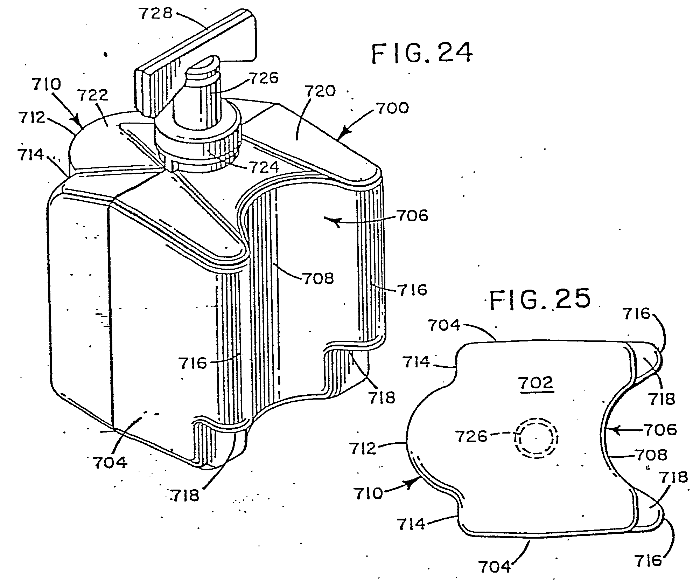

Referring to Figures 24 and 25, material supply container 700 is molded of high density polyethylene and has a bottom wall 702 joined to two pairs of opposed side walls. Side walls 704 are substantially flat and parallel, while side wall 706 comprises a central recess 708. Side wall 710, on the other hand, comprises a central protrusion 712 which is flanked by a pair of shoulders 714. Recess 708 is flanked by a pair of ridges 716 which extend less than the full height of the container to define shoulders 718. Top wall 720 comprises a sloped portion 722 adjacent protrusion 712. An annular collar 724 surrounds an outlet tube 726. A break-off closure 728 closes outlet tube 726.

As seen in Figures 1 through 5 and 19, this configuration enables supply container 700 to compactly nest around battery 502. Collar 724 functions to support container 700 in an inverted position on central flange 620 of lid 614 with outlet tube 726 extending through central aperture 616 and into fluid communication with material contained in reservoir 602. When in this position, shoulders 718 of container 700 are engaged by lateral projections 610 of arms 606 (see Figures 16 and 19).

An alternative embodiment of the supply container is illustrated in Figures 28, 29 and 30. Container 900 is generally similar in capacity, size and shape to container 700 and is also nestable with others of the same type. However, container 900 is devoid of shoulders 718 because an alternative container engaging means in the form of two opposed dimples 902 are formed in the container wall. Dimples 902 are engaged by snap tabs 816 of lid 814, which firmly retain container 900 in position, as seen in Figures 26 and 27. Container 900 also is slightly thinner than container 700 so as to clear retaining projections 610. Of course, projections 610 serve no functional purpose when container 900 is used.

Operation

When container 700 (or 900) is installed in cavity 309, protrusion 712 and adjacent shoulders 714 cooperate with housing front wall 302 to define an airway 730 (see Figures 1 through 5) which direct air forced through the housing by fan 404 downwardly and across reservoir 602. Because the top wall 720 of container 700 is raised above reservoir lid 614 by means of collar 724, sloped portion 722 cooperates with the lid to define an air passage 732 of decreasing cross-section for air flowing across the reservoir. The velocity of the air flow in the vicinity of reservoir 602 is therefore increased, with a corresponding reduction in static pressure. This reduced pressure enhances the evaporation of material from the surface of the liquid in the reservoir. As the air passes across apertured reservoir lid 614, the vaporized material is entrained therein and is exhausted to the ambient through channel 622 and outlet 307. If the rate of evaporation is insufficient, an absorbent wick (not shown) may be partially immersed in the material in reservoir 602 and extend outwardly therefrom into the air passage 732.

Material supply container 700 will automatically replenish material evaporated from reservoir 602 when the material level drops below the open mouth of outlet tube 726. This will break the vacuum created in supply container 700 and permit air to enter the container and displace a dispensed quantity of fresh liquid material. The material level within reservoir 602 will be brought back up to the level of the open mouth of outlet tube 726, at which time a liquid seal is again effected to discontinue the flow of material out of container 700. The fresh material is dispensed from supply container 700 directly onto the surface of the liquid in reservoir 602. Hence, the surface is continually renewed as the material evaporates, thereby preventing stagnation and encrustation of the surface, and insuring that the rate of evaporation remains substantially uniform. Because supply container 700 is air-tight, a supply of fresh material for replenishment is insured.

Although the present invention has been illustrated in terms of a preferred embodiment, it will be obvious to one of ordinary skill in the art that numerous modifications may be made without departing from the true spirit and scope of the invention which is to be limited only by the appended claims. For example, although a liquid air freshening material has been disclosed, it is clear that the dispenser of the invention can be used to dispense any vaporizable fluent substance, including liquid, powder and granular fluent substances. Furthermore, while a battery-powered dispenser has been described, it is envisioned that the dispenser could be powered by line current either directly to a motor designed to operate at higher voltage, or through a transformer which may replace the battery in the battery tray.

**Claims**

1. A method of emitting a vaporized material into the ambient at a controlled, substantially uniform rate from a substantially open reservoir (600) containing an exposed liquid quantity of said material, said quantity being replenished from a replaceable, air-tight supply container of fresh liquid material (700) removably supported above and in close proximity to said reservoir (600), thereby forming a small gap (732) between the body of said container (700) and said reservoir (600), said reservoir (600) and said container (700) being located within a housing (300) having air inlet means (124, 205, 210) and air outlet means (307), characterized by the steps of: producing and directing a forced flow of ambient air within said housing (300) through said gap (732) past and close to the surface of the liquid in the reservoir (600) to effect vaporization of said liquid from said surface and directly entrain the vaporized material in said air flow; and continually and automatically replenishing material evaporated from said reservoir (600) by discharging liquid material directly onto the surface of the liquid in the reservoir (600) from said container (700) to renew said surface and prevent stagnation thereof.

2. A method according to claim 1 wherein said supply container (700) has a downwardly directed outlet (726) in sealed fluid communication with said liquid surface, and the step of replenishing said material comprising dispensing fresh liquid from said container (700) onto said surface when the liquid seal between said container outlet (726) and said surface is broken by a drop in the surface level due to depletion of the liquid in the reservoir (600) by vaporization thereof.

3. A dispenser for emitting a vaporized material into the ambient at a controlled, substantially uniform rate comprising a housing (300) having air inlet means (124, 205, 210) and air outlet means (307), a substantially open reservoir (600) within said housing (300) for containing an exposed quantity of liquid material to be vaporized, an air-tight supply container (700) for continually and automatically replenishing material evaporated from said reservoir (600), support means (620) for removably supporting the supply container (700) within said housing (300), and air circulation means (400) for effecting a forced flow of ambient air through said dispenser, characterized in that air channelling means (712, 724) is provided for directing said flow of air past and close to the surface of the liquid in said reservoir (600) to effect vaporization of said liquid from said surface and directly entrain the vaporized material in said air-flow, and said supply container (700) discharges fresh liquid directly onto the surface of the liquid in said reservoir (600) to renew said surface and prevent stagnation thereof.

4. A dispenser according to claim 3 wherein said support means (620) supports said supply container (700) above said reservoir (600), and said air channelling means (712, 714) directs said air flow between said reservoir (600) and said container (700).

5. A dispenser according to claim 4 wherein said supply container (700) has a downwardly directed outlet (726) in sealed fluid communication with said liquid surface, through which outlet (726) fresh liquid is dispensed from said container (700) directly onto said surface whenever the liquid seal between said container outlet (726) and said liquid surface is broken by a drop in the surface level due to depletion of the liquid in the reservoir (600) by vaporization thereof.

6. A dispenser according to claim 3 further characterized in that said air channelling means (712, 724), is located on said container (700).

7. A dispenser according to claim 3 wherein said air channelling means comprises a protrusion (712) formed on the exterior of said supply container (700) which cooperates with said housing (300) to define an airway (730) for directing said air flow toward said reservoir (600).

8. A dispenser according to claim 5 or 7 wherein said supply container (700) comprises a side wall (704), a top wall (720) and a material outlet (726) formed in said top wall (720), said protrusion (712) being formed as part of said side wall (704), and said support means comprises a protruding shoulder (724) surrounding said material outlet.

9. A dispenser according to claim 8 wherein said top wall (720) has a sloping portion (722) which slopes downwardly and away from said shoulder (724), said container (700) being supported on said shoulder (724) in an inverted position above said reservoir (600), said air passage being defined in part by said sloping portion (722) and having a decreasing cross-section for air approaching said reservoir (600), whereby said air flow is caused to accelerate as it approaches said reservoir (600), thereby resulting in a drop in static air pressure which enhances vaporization of said material from said reservoir (600).

10. A dispenser according to claim 3 wherein said support means comprises lid means (614) supported on and overlying at least a portion of said reservoir (600) for supporting said supply container (700) and providing free passage for vaporized material leaving said reservoir (600).

11. A dispenser according to claim 10 wherein said reservoir (600) has retaining means (606) which is cooperable with said lid means (614) to retain said supply container (700) in position above said reservoir (600).

12. A dispenser according to claim 11 wherein said retaining means (606) is integrally formed with said reservoir (600).

13. A dispenser according to claim 12 wherein said retaining means comprises at least one arm (606) which engages said supply container (700) to retain it in position.

14. A dispenser according to claim 13 wherein said arm (606) extends generally upwardly from said reservoir and has a lateral projection (610) which engages a portion (718) of said supply container (700).

15. A dispenser according to claim 14 wherein said supply container (700) has a top wall (720), a material outlet (726) formed in said top wall (720) and a lower shoulder (718), said container (700) being supported in an inverted position with said lateral projection (610) engaging said lower shoulder (718).

16. A dispenser according to claim 12 wherein said container (700) has a wall with at least one dimple (902) formed therein, said retaining means comprising at least one snap tab (816) which engages said dimple (902).

17. A dispenser according to claim 16 wherein said wall has a pair of opposed dimples (902) and said retaining means comprises a pair of opposed snap tabs (816) which engage said dimples (902) to retain said container (700) therebetween.

18. A dispenser according to claim 10 wherein said lid means (614) has at least one aperture (618) formed therein through which said vaporized material can escape from said reservoir (600).

19. A dispenser according to claim 18 wherein said lid means (614) has a plurality of apertures (618) formed therein, and said supply container (700) has an outlet tube (726) which extends through one of said apertures (616).

20. A dispenser according to claim 19 wherein said outlet tube (926) is surrounded by a shoulder (724) which supports said container (700) on said lid means (614) in spaced relation thereto.

21. A dispenser according to claim 20 wherein said material is a fluent material and said outlet tube (726) has an open free end disposed in fluid communication with the material in said reservoir (600), whereby a substantially constant level of material in said reservoir (600) is maintained at the free end of said outlet tube (926).

22. A dispenser according to claim 3 wherein said air circulation means is electrically driven by a battery (502) electrically connected thereto, further characterized in that said container (700) has an outer wall (706) with a recess (708) formed therein, and said battery (502) is nested within said recess (708).

23. A dispenser according to claim 22 wherein said battery (502) is removably supported in battery support means (216) and said container (700) is removably supported independently of said battery (502) whereby said container (700) may be removed and replaced without removing said battery (502).

24. A dispenser according to claim 23 wherein said battery support means (216) comprises a pair of electrical contacts (504, 512) connected to said air circulation means (400).

25. A dispenser according to claim 24 wherein said air circulation means (400) comprises a motor-driven fan (402, 404), said motor (402) having a pair of motor terminals (408, 410), and each of said electrical contacts (504, 512) comprises a spring lead having one end portion in contact with one of the terminals of said battery (502) and the other end portion in contact with one of said motor terminals (408, 410).

26. A dispenser according to claim 25 wherein one of said motor terminals is formed as a hook (408), the end portion (516) of one of said spring leads (510) being retained by said hook (408) and being disengageable therefrom to switch off said motor (402).

27. A dispenser according to claim 26 wherein said end portion (516) of one of said spring leads (510) which is retained by said hook (408) comprises a helical coil (514) having a laterally extending tip (516) which is biased away from said hook by said helical coil (514).

28. A dispenser according to claim 27 further comprising a post (221) mounted adjacent to said motor (402), said helical coil (514) being supported on said post (221) and movable therearound when said tip (516) is moved into or out of engagement with said hook (408).

29. A dispenser according to claim 6 wherein said container (700) is disposed intermediate said air circulation means (400) and said reservoir (600) so that substantially all of the air flowing through said housing (300) is channeled by said air channelling means (712, 724).

30. A dispenser according to claim 29 wherein said air circulation means (400), said supply container (700) and said reservoir (600) are vertically stacked with said air circulation (400) means disposed at the top and said reservoir disposed (600) at the bottom.

31. A dispenser according to claim 6 further characterized in that said housing (300) has a viewing window (326) and said reservoir (600) has a transparent portion (604) in registry with said window (326) so that the condition of the material within said reservoir (600) can be ascertained externally of said housing (300).

## Patentansprüche

1. Verfahren zur Einbringung eines verdampften Materials in die Umgebung in einer kontrollierten, im wesentlichen gleichmäßigen Rate aus einem im wesentlichen offenen Reservoir (600), das eine offenliegende Menge dieses Materials enthält, die aus einem auswechselbaren, luftdichten Versorgungsbehälter mit frischem, flüssigem Material (700) nachgefüllt wird, der lösbar und in enger Nähe zu dem Reservoir (600) gehalten ist, wodurch ein kleiner Spalt (732) zwischen dem Körper des Behälters (700) und dem Reservoir gebildet wird, wobei das Reservoir (600) und der Behälter (700) in einem Gehäuse (300) untergebracht sind, das mit Lufteinlaßmitteln (124, 205, 210) und Luftauslaßmitteln (307) ausgestattet ist, gekennzeichnet durch die Schritte: Hervorrufen und Dirigieren eines voranbewegten Stromes von Umgebungsluft innerhalb des Gehäuses (300) durch den Spalt (732) hindurch und dicht über die Oberfläche der Flüssigkeit in dem Reservoir (600) zur Herbeiführung der Verdampfung der Flüssigkeit aus der Oberfläche und zur direkten Einbringung des verdampften Materials in den Luftstrom; und fortlaufendes und selbsttätiges Nachfüllen von Material, das aus dem Reservoir (600) verdampft ist, durch Aufgeben von flüssigem Material direkt auf die Flüssigkeitsoberfläche in dem Reservoir (600) aus dem Behälter (700) zur Erneuerung der Fläche und zur Verhütung ihres Stillstandes.

2. Verfahren nach Anspruch 1, bei dem der Versorgungsbehälter (700) einen abwärts gerichteten Auslaß (726) aufweist, der in dichtender Flüssigkeitsverbindung mit der Flüssigkeitsoberfläche steht, und bei dem der Schritt des Nachfüllens von Material die Ausgabe frischer Flüssigkeit aus dem Behälter (700) auf die Oberfläche umfaßt, wenn die Flüssigkeitsdichtung zwischen dem Behälterauslaß (726) und der Oberfläche in Folge eines Absinkens des Oberflächeniveaus aufgrund der Entleerung der Flüssigkeit in dem Reservoir (600) durch Verdampfung unterbrochen wird.

3. Verdunstungsspender zur Einbringung eines verdampften Materials in die Umgebung in einer kontrollierten, im wesentlichen gleichmäßigen Rate, mit einem Gehäuse (300), das Lufteinlaßmittel (124, 205, 210) und Luftauslaßmittel (307) enthält, mit einem im wesentlichen offenen Reservoir (600) innerhalb des Gehäuses (300) zur Aufnahme einer offenliegenden Menge von flüssigem, zu verdampfendem Material, einem luftdichten Versorgungsbehälter (700) zur fortlaufenden und automatischen Nachfüllung von Material, das aus dem Reservoir (600) verdampft ist, mit Unterstützungsmitteln (620) zur lösbaren Befestigung des Versorgungsbehälters (700) innerhalb des Gehäuses (300) und mit Luftzirkulationsmitteln (400) zur Herbeiführung eines voranbebewegten Stromes von Umgebungsluft durch den Spender, dadurch gekennzeichnet, daß Luftleitmittel (712, 724) zur Lenkung des Luftstromes an der Fläche vorbei und nah über Fläche der Flüssigkeit in dem Reservoir (600) hinweg vorgesehen sind, um eine Verdampfung der Flüssigkeit aus der Oberfläche zu bewirken und um das verdampfte Material direkt in den Luftstrom hineinzutragen, und daß der Vorratsbehälter (700) frische Flüssigkeit direkt auf die Oberfläche der Flüssigkeit in dem Reservoir (600) aufgibt, um die Oberfläche zu erneuern und deren Stillstand zu verhindern.

4. Verdunstungsspender nach Anspruch 3, bei dem die Unterstützungsmittel (620) den Versorgungsbehälter (700) oberhalb des Reservoirs (600) halten und bei dem die Luftleitmittel (712, 724) den Luftstrom zwischen das Reservoir (600) und den Behälter (700) lenken.

5. Verdunstungsspender nach Anspruch 4, bei dem der Versorgungsbehälter (700) einen abwärts gerichteten Auslaß (726) aufweist, der in flüssigkeitsdichter Verbindung mit der Flüssigkeitsoberfläche steht, wobei aus dem Auslaß (726) frische Flüssigkeit aus dem Behälter (700) direkt auf die Oberfläche aufgegeben wird, wann immer die Flüssigkeitsdichtung zwischen dem Behälterauslaß (726) und der Flüssigkeitsoberfläche in Folge eines Absinkens des Oberflächenniveaus aufgrund der Entleerung von Flüssigkeit in dem Reservoir (600) durch Verdampfung unterbrochen wird.

6. Verdunstungsspender nach Anspruch 3, dadurch gekennzeichnet, daß die Luftleitmittel (712, 724) an dem Behälter (700) angebracht sind.

7. Verdunstungsspender nach Anspruch 3, bei dem die Luftleitmittel einen Vorsprung (712) auf der Außenseite des Versorgungsbehälters (700) einschließen, der mit dem Gehäuse (300) zur Festlegung eines Luftweges (730) zusammenwirkt, um den Luftstrom in Richtung des Reservoirs (600) zu lenken.

8. Verdunstungsspender nach Anspruch 5 oder 7, bei dem der Vorsorgungsbehälter (700) eine Seitenwand (704), eine Oberseitenwand (720) und einen Materialauslaß (726) innerhalb der Oberseitenwand (720) enthält, wobei der Vorsprung (712) einen Teil der Seitenwand (704) bildet und die Unterstützungsmittel eine vorspringende Schulter (724) enthalten, die den Materialauslaß umgibt.

9. Verdunstungsspender nach Anspruch 8, bei dem die Oberseitenwand (720) einen abfallenden Abschnitt (722) enthält, der abwärts und weg von der Schulter (724) geneigt ist, bei dem der Behälter (700) auf der Schulter (724) in einer Überkopflage oberhalb des Reservoirs (600) gehalten ist, und bei dem der Luftweg zum Teil durch den abfallenden Abschnitt (722) gebildet wird und einen abnehmenden Querschnitt für das Reservoir (600) anströmende Luft aufweist, wodurch der Luftstrom bei der Annäherung an das Reservoir (600) beschleunigt wird und wodurch ein Abfall des statischen Luftdruckes herbeigeführt wird, der die Verdampfung des Materials aus dem Reservoir (600) verstärkt.

10. Verdunstungsspender nach Anspruch 3, bei dem die Unterstützungsmittel Deckelmittel (614) umfassen, die auf einem Teil des Reservoirs (600) ruhen und mindestens einen Teil des Reservoirs (600) überdecken, um den Versorgungsbehälter (700) zu halten und einen freien Durchgang für das verdampfte Material, das das Reservoir (600) verläßt, bereitzustellen.

11. Verdunstungsspender nach Anspruch 10, bei dem das Reservoir (600) Haltemittel (606) enthält, die mit den Deckelmitteln (614) zur Halterung des Versorgungsbehälter (700) in einer Lage über dem Reservoir (600) zusammenwirken.

12. Verdunstungsspender nach Anspruch 11, bei dem die Haltemittel (606) als integraler Bestandteil mit dem Reservoir (600) ausgeformt sind.

13. Verdunstungsspender nach Anspruch 12, bei dem die Haltemittel mindestens einen Arm (606) umfassen, der den Versorgungsbehälter (700) zur Halterung in seiner Lage erfaßt.

14. Verdunstungsspender nach Anspruch 13, bei dem der Arm (606) im wesentlichen aufwärts von dem Reservoir aufragt und seitliche Vorsprünge (610) trägt, die einen Abschnitt (718) des Versorgungsbehälters (700) erfassen.

15. Verdunstungsspender nach Anspruch 14, bei dem der Versorgungsbehälter (700) eine Oberseitenwand (720), einen Materialauslaß (726) innerhalb der Oberseitenwand (720) und eine untere Schulter (718) aufweist, wobei der Behälter (700) in einer Überkopflage gehalten ist, in der die seitlichen Vorsprünge (610) die untere Schulter (718) ergreifen.

16. Verdunstungsspender nach Anspruch 12, bei dem der Behälter (700) eine Wand mit mindestens einer darin eingeformten Vertiefung (902) aufweist, und bei dem die Haltemittel mindestens einen Rastnocken (816) enthalten, der in die Vertiefung (902) einrasten.

17. Verdunstungsspender nach Anspruch 16, bei dem die Wand ein Paar von sich gegenüberliegenden Vertiefungen (902) aufweist, und bei dem die Haltemittel ein Paar von sich gegenüberliegenden Rastnocken (816) enthalten, die in die Vertiefungen (902) einrasten, um den Behälter (700) dazwischen zu halten.

18. Verdunstungsspender nach Anspruch 10, bei dem die Deckelmittel (614) mindestens eine Öffnung (618) aufweisen, durch die das verdampfte Material aus dem Reservoir (600) austreten kann.

19. Verdunstungsspender nach Anspruch 18, bei dem die Deckelmittel (614) eine Mehrzahl von Öffnungen (618) aufweisen, und bei dem der Versorgungsbehälter (700) ein Auslaßrohr (726) trägt, das durch eine der Öffnungen (616) hindurchragt.

20. Verdunstungsspender nach Anspruch 19, bei dem das Auslaßrohr (926) von einer Schulter (724) umgeben ist, die den Behälter (700) auf den Deckelmitteln (614) in einem Abstand dazu hält.

21. Verdunstungsspender nach Anspruch 20, bei dem das Material ein flüssiges Material ist und bei dem das Auslaßrohr (726) ein offenes, freies Ende aufweist, das in flüssiger Verbindung mit dem Material in dem Reservoir (600) steht, wodurch ein im wesentlichen konstantes Niveau von Material in dem Reservoir (600) an dem freien Ende des Auslaßrohres (926) beibehalten wird.

22. Verdunstungsspender nach Anspruch 3, bei dem die Luftumwälzmittel mit Hilfe einer daran elektrisch angeschlossenen Batterie (502) elektrisch angetrieben sind, dadurch gekennzeichnet, daß der Behälter (700) eine äußere Wand (706) mit einem darin eingeformten Rücksprung (708) enthält, und daß die Batterie (502) innerhalb des Rücksprungs (708) eingebettet ist.

23. Verdunstungsspender nach Anspruch 22, bei dem die Batterie (502) lösbar in Batterie-Haltemitteln (216) gehalten ist, und bei dem der Behälter (700) lösbar und unabhängig von der Batterie (502) gehalten ist, wobei der Behälter (700) herausgenommen und ersetzt werden kann, ohne die Batterie (502) zu entnehmen.

24. Verdunstungsspender nach Anspruch 23, bei dem die Batterie-Haltemittel (216) ein Paar von elektrischen Kontakten (504, 512) enthalten, die an die Luftumwälzmittel (400) angeschlossen sind.

25. Verdunstungsspender nach Anspruch 24, bei dem die Luftumwälzmittel (400) einen motorbetriebenen Lüfter (402, 404) beinhalten, bei dem der Motor (402) ein Paar Motoranschlüsse (408, 410) aufweist, und bei dem jeder der elektrischen Kontakte (504, 512) eine Federleitung aufweist, von der der eine Endabschnitt mit einem der Anschlüsse der Batterie (502) und der andere Endabschnitt in Verbindung mit einem der Motoranschlüsse (408, 410) steht.

26. Verdunstungsspender nach Anspruch 25, bei dem einer der Motoranschlüsse als Haken (408) ausgebildet ist, und bei dem der Endabschnitt (516) einen der Federleitungen (510) durch den Haken (408) gehalten und davon trennbar ist, um den Motor (402) auszuschalten.

27. Verdunstungsspender nach Anspruch 26, bei dem der Endabschnitt (516) der einen der Federleitungen (510), der von dem Haken (408) zurückgehalten ist, eine wendelförmige Feder (514) enthält, die eine seitlich hervorstehende Spitze (516) aufweist, die von der wendelförmigen Feder (514) weg von dem Haken vorgespannt ist.

28. Verdunstungsspender, nach Anspruch 24, mit einem unmittelbar neben dem Motor (402) befestigten Pfosten (221), an dem die wendelförmige Feder (514) gehalten ist und um den sie beweglich ist, wenn die Spitze (516) in oder außer Eingriff mit dem Haken (408) gebracht wird.

29. Verdunstungsspender nach Anspruch 6, bei dem der Behälter (700) zwischen den Luftumwälzmitteln (400) und dem Reservoir (600) so angeordnet ist, daß im wesentlichen die gesamte Luft, die durch das Gehäuse (300) strömt, von den Luftleitmitteln (712, 724) gelenkt werden.

30. Verdunstungsspender nach Anspruch 29, bei dem die Luftumwälzmittel (400), der Versorgungsbehälter (700) und das Reservoir (600 vertikal übereinander angeordnet sind, wobei die Luftzirkulationsmittel (400) oben und das Reservoir (600) unten angeordnet sind.

31. Verdunstungsspender nach Anspruch 6, dadurch gekennzeichnet, daß das Gehäuse (300) ein Beobachtungsfenster (326) enthält, und daß das Reservoir (600) eine durchsichtige Stelle (604) in Höhe des Fenster (326) aufweist, so daß der Zustand des Materials in dem Reservoir (600) von außerhalb des Gehäuses (300) festgestellt werden kann.

**Revendications**

1. Procédé pour dispenser un produit évaporé dans l'atmosphère ambiante à une vitesse réglée essentiellement uniforme à partir d'un réservoir (600) essentiellement ouvert contenant une quantité du produit liquide exposé à l'air, cette quantité étant complétée à partir d'un conteneur d'approvisionnement du produit liquide frais (700), qui est remplaçable, étanche à l'air et supporté de façon amovible au-dessus et à proximité immédiate dudit réservoir (600), en formant ainsi un faible interstice (732) entre le corps dudit conteneur (700) et ledit réservoir (600), ledit réservoir (600) et ledit conteneur (700) étant situés à l'intérieur d'un boîtier (300) comportant un dispositif d'entrée d'air (124, 205, 210) et un dispositif de sortie d'air (307), caractérisé par les phases opératoires consistant à produire et diriger un écoulement forcé d'air ambiant à l'intérieur dudit boîtier (300) à travers lesdits interstices (732) devant et à proximité de la surface du liquide situé dans le réservoir (600) de manière à réaliser l'évaporation du liquide à partir de ladite surface et à entraîner directement le produit évaporé dans ledit écoulement d'air, et à compléter de façon continue et automatique le produit évaporé hors dudit réservoir (600) par refoulement du produit liquide directement à la surface du liquide situé dans le réservoir (600), à partir dudit conteneur (700), de manière à renouveler ladite surface et à empêcher une stagnation au niveau de cette dernière.

2. Procédé selon la revendication 1, selon lequel ledit conteneur d'approvisionnement (700) possède une sortie (726) dirigée vers le bas et qui est en communication fluidique étanche avec ladite surface liquide, et selon lequel, la phase opératoire de remplissage complémentaire du produit inclut la délivrance de liquide frais depuis ledit conteneur (700) sur ladite surface lorsque l'étanchéité liquide entre ladite sortie (726) du conteneur et ladite surface est interrompue par une baisse du niveau de la surface, due à une diminution du liquide dans le réservoir (600) par suite de l'évaporation de ce liquide.

3. Distributeur destiné à dispenser un produit évaporé dans l'air ambiant, à une vitesse réglée essentiellement uniforme, comprenant un boîtier (300) possédant un dispositif d'entrée d'air (124, 205, 210) et un dispositif de sortie d'air (307), un réservoir (600) essentiellement ouvert situé à l'intérieur du boîtier (300) de manière à contenir une quantité de produit liquide exposé à l'air et devant être évaporé, un conteneur d'approvisionnement (700) étanche à l'air et servant à compléter de façon continue et automatique le remplissage du produit évaporé hors dudit réservoir (600), un dispositif de support (620) permettant de supporter de façon amovible le conteneur d'approvisionnement (700) à l'intérieur dudit boîtier (300), et un dispositif de circulation de l'air (400) servant à réaliser un écoulement forcé de l'air ambiant à travers ledit distributeur, caractérisé en ce que des moyens de canalisation de l'air (712, 724) sont prévus de manière à diriger ledit écoulement d'air devant et à proximité de la surface du liquide situé dans ledit réservoir (600) de manière à réaliser l'évaporation dudit liquide à partir de ladite surface et à entraîner directement le produit évaporé dans ledit écoulement d'air, et que ledit conteneur d'approvisionnement (700) refoule du liquide frais directement à la surface du liquide dans ledit réservoir (600) de manière à renouveler ladite surface et à empêcher une stagnation au niveau de cette dernière.

4. Distributeur selon la revendication 3, dans lequel ledit dispositif de support (620) soutient ledit conteneur d'approvisionnement (700) au-dessus dudit réservoir (600) et que lesdits moyens de canalisation de l'air (712, 724) dirigent ledit écoulement d'air entre ledit réservoir (600) et ledit conteneur (700).

5. Distributeur selon la revendication 4, dans lequel ledit conteneur d'approvisionnement (700) possède une sortie (726) dirigée vers le bas et qui est en communication fluidique. étanche avec ladite surface liquide et par par l'intermédiaire de laquelle le liquide frais est distribué à partir du conteneur (700) directement au niveau de ladite surface toutes les fois que l'étanchéité liquide entre ladite sortie (726) du conteneur et ladite surface liquid est interrompue par une chute du niveau de la surface par suite de la diminution du liquid dans le réservoir (600) sous l'effet de l'évaporation du liquide.

6. Distributeur selon la revendication 3, caractérisé en outre en ce que lesdits moyens de canalisation de l'air (712, 724) sont situés sur ledit conteneur (700).

7. Distributeur selon la revendication 3, dans lequel lesdits moyens de canalisation de l'air comprennent une partie saillante (712) ménagée à l'extérieur dudit conteneur d'approvisionnement (700) et qui coopère avec ledit boîtier (300) de manière à définir un passage d'air (730) servant à diriger ledit écoulement d'air vers ledit réservoir (600).

8. Distributeur selon la revendication 5 ou 7, dans lequel ledit conteneur d'approvisionnement (700) comporte une paroi latérale (704), une paroi supérieure (720) et une sortie du produit (726) ménagée dans ladite paroi supérieure (720), ladite partie saillante (712) étant réalisée en tant que partie de ladite paroi latérale (704), dans lequel ledit liquide de support comporte un épaulement saillant (724) entourant ladite sortie du produit.

9. Distributeur selon la revendication 8, dans lequel ladite paroi supérieure (720) possède une partie inclinée (722) qui descend obliquement en s'écartant dudit épaulement (724), ledit conteneur (700) étant supporté par ledit épaulement (724) dans une position renversée au-dessus dudit réservoir (600), ledit passage d'air étant défini par la partie inclinée (722) et possédant une section transversale décroissante pour l'air se rapprochant dudit réservoir (600), ce qui a pour conséquence une accélération dudit écoulement d'air lorsque ce dernier se rapproche dudit réservoir (600), avec pour conséquence une chute de la pression statique de l'air, qui accroît l'évaporation dudit produit à partir dudit réservoir (600).

10. Distributeur selon la revendication 3, dans lequel ledit dispositif de support comporte un dispositif formant couvercle (614) porté par et recouvrant au moins une partie dudit réservoir (600) de manière à soutenir ledit conteneur d'approvisionnement (700) et fournissant un passage libre pour le produit évaporé quittant ledit réservoir (600).

11. Distributeur selon la revendication 10, dans lequel ledit réservoir (600) comporte un dispositif de retenue (606) qui peut coopérer avec ledit dispositif formant couvercle (614) pour maintenir ledit conteneur d'approvisionnement (700) en position au-dessus dudit réservoir (600).

12. Distributeur selon la revendication 11, dans lequel ledit dispositif de retenue (606) est formé d'un seul tenant avec ledit réservoir (600).

13. Distributeur selon la revendication 12, dans lequel ledit dispositif de retenue comporte au moins un bras (606) qui engrène avec ledit conteneur d'approvisionnement (700) pour le maintenir en position.

14. Distributeur selon la revendication 13, dans lequel ledit bras (606) s'étend dans son ensemble vers le haut à partir dudit réservoir et possède une partie saillante latérale (610) qui engrène avec une partie (718) dudit conteneur d'approvisionnement (700).

15. Distributeur selon la revendication 14, dans lequel ledit conteneur d'approvisionnement (700) possède une paroi supérieure (720), une sortie du produit (726) ménagée dans ladite paroi supérieure (720) et un épaulement inférieur (718), ledit conteneur (700) étant soutenu dans une position renversée par ladite partie saillante latérale (610) engrenant avec ledit épaulement inférieur (718).

16. Distributeur selon la revendication 12, dans lequel ledit conteneur (700) possède une paroi, dans laquelle au moins un renfoncement (902) est ménagé, ledit dispositif de retenue comportant au moins une languette d'encliquetage brusque (816) qui engrène avec ledit renfoncement (902).

17. Distributeur selon la revendication 16, dans lequel ladite paroi possède deux renfoncements opposés (902) et ledit dispositif de retenue comporte deux languettes opposées d'encliquetage brusque (818), qui engrènent avec lesdits renfoncements (902) de manière à maintenir ledit conteneur (700) entre elles.

18. Distributeur selon la revendication 10, dans lequel ledit dispositif formant couvercle (614) comporte au moins une ouverture (618) à travers laquelle ledit produit évaporé peut s'échapper hors dudit réservoir (600).

19. Distributeur selon la revendication 18, dans lequel ledit dispositif formant couvercle (614) comporte plusieurs ouvertures (618) et dans lequel ledit conteneur d'approvisionnement (700) possède un tube de sortie (726) qui s'étend à travers l'une desdites ouvertures (616).

20. Distributeur selon la revendication 19, dans lequel ledit tube de sortie (726) est entouré par un épaulement (724) qui soutient ledit conteneur (700) sur ledit dispositif formant couvercle (614), tout en en étant distant.

21. Distributeur selon la revendication 20, dans lequel ledit produit est une substance fluide et ledit tube de sortie (726) possède une extrémité libre ouverte qui est en communication fluidique avec le produit situé dans le réservoir (600), ce qui a pour effet qu'un niveau essentiellement constant du produit dans ledit réservoir (600) est maintenu au niveau de l'extrémité libre du tube de sortie (726).

22. Distributeur selon la revendication 3, dans lequel ledit dispositif de circulation de l'air est entraîné électriquement par une pile (502), qui lui est raccordée électriquement, et caractérisé en outre en ce que ledit conteneur (700) possède une paroi extérieure (706), dans laquelle est ménagé un renfoncement (708), et que ladite pile (702) est logée dans ledit renfoncement (708).

23. Distributeur selon la revendication 22, dans lequel ladite pile (502) est portée de façon amovible dans le dispositif de support de pile (216) et ledit conteneur (700) est supporté de façon amovible et indépendamment de ladite pile (502), ce qui a pour effet que ledit conteneur (700) peut être retiré et remis en place sans retirer ladite pile (502).

24. Distributeur selon la revendication 23, dans lequel ledit dispositif de support de pile (216) comporte deux contacts électriques (504, 512) raccordés audit dispositif de circulation de l'air (400).

25. Distributeur selon la revendication 24, dans lequel ledit dispositif de circulation de l'air (400) comporte un ventilateur (402, 404) entraîné par un moteur (402) possédant deux bornes (408, 410), et chacun desdits contacts électriques (504, 512) comporte un conducteur formant ressort dont une extrémité est en contact avec l'une des bornes de la pile (502) et dont l'autre extrémité est en contact avec l'une desdites bornes (408, 410) du moteur.

26. Distributeur selon la revendication 25, dans lequel l'une desdites bornes du moteur est réalisée sous la forme d'un crochet (408), l'extrémité (516) de l'un desdits conducteurs formant ressort (510) étant retenue par ledit crochet (408) et pouvant être dégagée de ce dernier de manière à débrancher ledit moteur (402).

27. Distributeur selon la revendication 26, dans lequel ladite extrémité (516) de l'un desdits conducteurs formant ressort (510), qui est retenue par ledit crochet (408), comporte un enroulement hélicoïdal (514) possédant une pointe (516) s'étendant latéralement et qui est écartée dudit crochet par ledit enroulement hélicoïdal (514).

28. Distributeur selon la revendication 27 comportant en outre un téton (221) monté au voisinage dudit moteur (402), ledit enroulement hélicoïdal (514) étant porté par ledit téton (221) et étant enroulé de façon amovible autour de ce dernier lorsque ladite pointe (516) est amenée en et hors de contact avec ledit crochet (408).

29. Distributeur selon la revendication 6, dans lequel ledit conteneur (700) est disposé entre ledit dispositif de circulation de l'air (400) et ledit réservoir (600) de telle sorte qu'essentiellement tout l'air circulant en travers ledit boîtier (300) est canalisé par ledit dispositif de canalisation de l'air (712, 724).

30. Distributeur selon la revendication 29, dans lequel ledit dispositif de circulation de l'air (400), ledit conteneur d'approvisionnement (700) et ledit réservoir (600) sont empilés verticalement, ledit dispositif de circulation de l'air (400) étant disposé à la partie supérieure et ledit réservoir (600) étant disposé à la partie inférieure.

31. Distributeur selon la revendication 6, caractérisé en outre en ce que ledit boîtier (300)

23 **0 019 626** 24

possède une voyant (326) et que ledit réservoir (600) possède une partie transparente (604) alignée avec ledit voyant (326) de telle sorte que l'on peut constater, à l'extérieur dudit boîtier (300), l'état du produit à l'intérieur dudit réservoir (600).

FIG.1

0 019 626

0 019 626

FIG. 2

2

FIG. 3

FIG. 5

FIG.4

FIG.6

FIG.7

FIG. 8

FIG.9

FIG.10

0019 626

FIG.11

FIG.12

FIG.13

FIG.14

FIG.15

7

FIG. 17

FIG. 18

FIG. 16

FIG. 19

FIG. 20 FIG. 21 FIG. 22 FIG. 23

FIG.24

FIG.25

FIG.26

900

902

28

816

814

602

606

28

606

900

606

816

902

902

816

814

618

602

FIG. 27

FIG.28

FIG.29

FIG.30